# FASCICULE DE BREVET EUROPEEN

(11) **EP 1 006 947 B1**
(45) Date de publication et mention de la délivrance du brevet: **28.04.2004**
(21) Numéro de dépôt: 97947092.9
(22) Date de dépôt: 19.11.1997
(51) Int. Cl.: A61F 2/08

(54) **LIGAMENT PROTHETIQUE PRE-ORIENTE ET PROCEDE DE CONFECTION**
VORORIENTIERTE SEHNENBANDPROTHESE SOWIE HERSTELLUNGSVERFAHREN
PRE-ADJUSTED PROSTHETIC LIGAMENT AND METHOD OF MANUFACTURE

(30) Priorité: 20.11.1996 FR 9614263
(43) Date de publication de la demande: 14.06.2000
(73) Titulaire: Laboureau, Jacques-Philippe, F-21000 Dijon (FR)
(72) Inventeur: LABOUREAU, Jacques-Philippe, F-21000 Dijon (FR); BRULEZ, Bernard, F-52400 Bourbonne les Bains (FR)
(74) Mandataire: Bruder, Michel
(86) Numéro de dépôt international: PCT/FR1997/002082
(87) Numéro de publication internationale: WO 1998/022046

(56) Documents cités:
- EP-A- 0 561 710
- DE-A- 3 923 580
- FR-A- 2 651 994
- FR-A- 2 697 151
- US-A- 5 263 984

## Description

L'invention concerne un ligament prothétique lévogyre ou dextrogyre pour le remplacement d'un ligament articulaire biologique ainsi que plusieurs procédés de confection de tels ligaments pré-orientés ; ces ligaments lévogyres ou dextrogyres sont notamment utilisables dans la plastie du genou pour le remplacement des ligaments croisés antérieurs ou postérieurs, qu'il s'agisse d'une articulation gauche ou droite.

On connaissait déjà un certain nombre de ligaments artificiels destinés au remplacement de ligaments articulaires. Parmi ceux-ci il existe des ligaments obtenus par pliage ou roulage convolute de bandes de textile d'un matériau biocompatible, tissées ou tricotées de manière à laisser libre ou, au contraire à lier entre-eux, les fils techniques longitudinaux du ligament.

C'est en particulier le cas des ligaments décrits dans le brevet français FR-2.697.151 au nom du demandeur qui, ajoutant aux qualités dynamométriques élevées des prothèses antérieures, visent à améliorer encore la réponse des ligaments aux sollicitations auxquelles ils sont soumis une fois implantés dans les articulations en s'approchant au plus près de la géométrie et de la configuration des ligaments anatomiques. A cet effet, il était proposé un ligament artificiel comportant deux parties extrêmes intra-osseuses et une partie intermédiaire intra-articulaire caractérisé en ce que la partie intra-articulaire était formée de deux cordons indépendants de fibres longitudinales obtenus par roulage convolute d'au moins une laize tricotée, partiellement fendue en sa partie médiane sur une longueur au moins égale à celle de la partie intra-articulaire, les deux parties intra-osseuses étant tordues d'au moins un quart de tour l'une par rapport à l'autre lorsque le ligament était en place dans l'articulation.

On sait bien en effet, qu'un tel ligament présente des qualités liées à la structure en deux cordons procurant notamment pour le remplacement du ligament croisé antérieur du genou, un avantage biomécanique incontestable puisqu'il est bien établi que le ligament croisé antérieur subi une torsion supérieure à 45° lorsque le genou passe de sa position maximale de flexion à sa position maximale d'extension ; ainsi en implantant un ligament artificiel pré-orienté dans le même sens que ce mouvement naturel de torsion entre le tibia et le condyle fémoral interne, sur le genou en flexion, on peut faire en sorte que, en position d'extension du genou, les deux cordons formant le ligament se détordent et présentent alors une structure parfaitement parallèle.

Cette disposition permet alors aux fils longitudinaux constituant l'âme technique du ligament de se retrouver parallèles entre-eux lorsque le genou est proche de l'extension c'est-à-dire dans la position où il est habituellement traumatisé lors des entorses ; il se produit en fait un recrutement progressif des fils lors des efforts de tension réalisant alors un véritable système amortisseur reproduisant celui de l'anatomie.

Toutefois on sait que cet effet n'est vraiment sensible sur un ligament artificiel que dans la mesure où un nombre suffisant de fils longitudinaux peuvent être mis en action d'emblée en entourant au plus près la fibre neutre isométrique, ce qui implique une construction prothétique comportant le plus grand nombre de fils dans le plus petit volume cylindrique; il est rappelé au passage, que la fibre neutre d'un ligament est celle qui passe par l'axe isométrique joignant les points isométriques des os de l'articulation concernée.

Or, on comprend facilement que dans une configuration en double faisceau telle que proposée dans le document antérieur, il soit difficile de recruter d'emblée un nombre suffisant de fils longitudinaux autour de la fibre neutre.

Il est alors apparu qu'un ligament artificiel tel que décrit dans le brevet français FR-2.688.690 au nom du même demandeur, caractérisé en ce que sa partie intra-articulaire est uniquement formée de fils adjacents non liés entre-eux, pourrait avantageusement procurer cet effet ; selon les enseignements de ce document antérieur le ligament proposé est réalisé à partir d'une laize préservant le parallélisme des fils adjacents qui peuvent par conséquent supporter les efforts appliqués aux ligaments implantés, suivant leur direction longitudinale propre.

Du fait de sa structure intra-articulaire non tissée, non tricotée et plus généralement non tramée, il est possible d'augmenter sensiblement le nombre de fils actifs tout en conservant le même encombrement qu'un ligament tricoté ou tissé sur toute sa longueur ; on peut ainsi pratiquement doubler le nombre de fils actifs du ligament.

En outre, cette disposition plus compacte des fils actifs permet de rapprocher ces derniers de la "fibre neutre" du ligament.

L'inconvénient d'un tel ligament dit à "*fils libres*", est de ne pas pouvoir être naturellement contourné sur lui-même dans sa zone médiane intra-articulaire, à l'instar du ligament double faisceaux décrit dans le brevet français FR-2.697.151 déjà cité.

La présente invention a justement pour objet de proposer un ligament à "*fils libres*" présentant naturellement une torsion sur lui-même entre les deux extrémités de la zone médiane intra-articulaire.

A cet égard, il est proposé selon l'invention un procédé de confection d'un ligament prothétique pour le remplacement d'un ligament articulaire naturel, comprenant entre deux parties extrêmes une partie médiane seulement formée par un écheveau de fils dits "actifs" mono ou multi-filamentaires dont le titre est inférieur ou égal à 1200 décitex, longitudinaux, adjacents et non transversalement liés entre-eux caractérisé en ce que préalablement au montage du ligament, on donne une torsion longitudinale à chaque fil actif dont on choisit le sens de torsion, horaire ou anti-horaire, pour former respectivement un ligament prothétique pour articulation droite ou articulation gauche, les extrémités des fils actifs étant maintenues ensemble par tous moyens appropriés sur une longueur suffisante pour constituer les parties extrêmes du ligament prothétique destinées à être insérées dans les tunnels osseux de l'articulation.

On comprend bien tous les avantages d'un tel procédé puisqu'il suffit de la manière qui sera développée plus loin d'imprimer une torsion suffisante à chaque fil actif pour que le couple de torsion qui en résulte crée au total un vrillage naturel du ligament sur lui-même d'au moins un huitième de tour entre les deux extrémités, de la zone médiane active, correspondant en pratique à une torsion optimale, du moins lorsque les tunnels d'insertion osseuse sont idéalement situés.

Sachant en outre que sur un genou fléchi à 90°, les deux faisceaux du ligament croisé antérieur biologique sont vrillés dans le sens horaire pour le genou droit, et dans le sens anti-horaire pour le genou gauche, et conformément à une caractéristique essentielle de l'invention, on choisira avantageusement un sens de torsion des fils actifs de la prothèse, horaire ou anti-horaire, déterminant respectivement un ligament prothétique pour une articulation droite ou une articulation gauche.

D'autres caractéristiques et avantages ressortiront mieux de la description qui va suivre, de plusieurs procédés de torsion des fils actifs selon qu'il est utilisé, au moins dans la partie intra-articulaire du ligament prothétique, un écheveau de fils mono-filamentaires ou multi-filamentaires, donnée à titre d'exemples non limitatifs en référence aux dessins sur lesquels :
- la figure 1 représente schématiquement les étapes principales d'une première variante du procédé de torsion des fils actifs dans une configuration mono-filamentaire, ou multi-filamentaires continus et parallèles.
- la figure 2 représente deux schémas de fils câblés aptes à procurer une torsion naturelle à chaque assemblage soit dans le sens horaire (schéma S), soit dans le sens anti-horaire (schéma Z),
- la figure 3 est une vue en élévation de ligaments prothétiques : lévogyre (3a) dextrogyre (3b) et lévogyre à deux enroulements convolutes (3c).

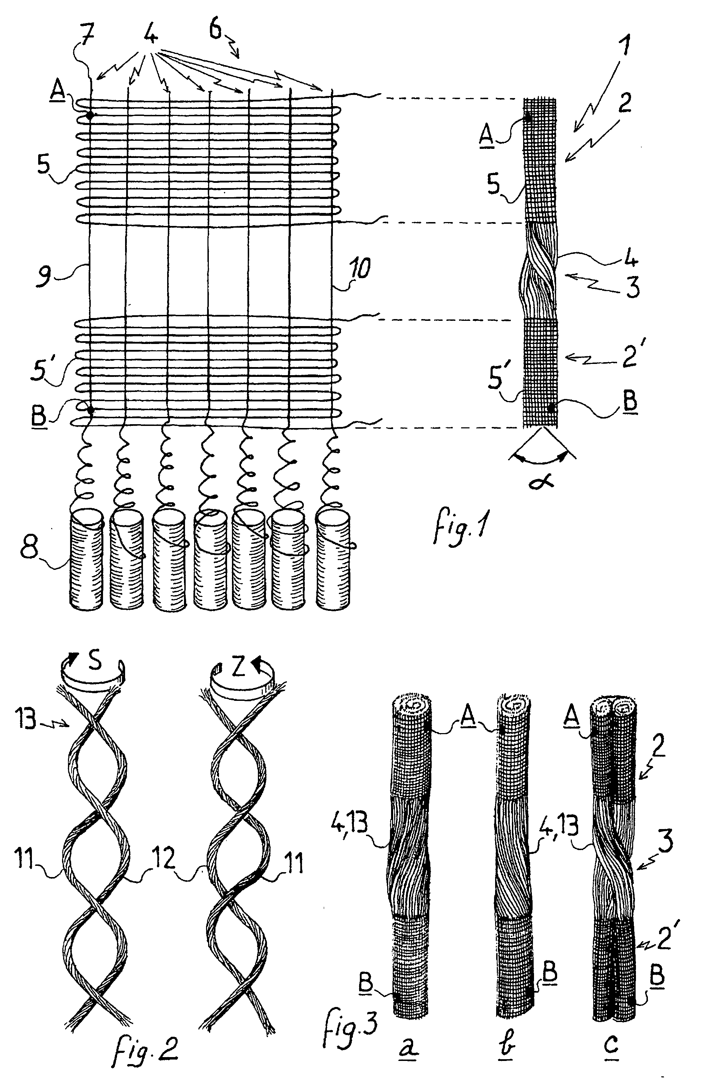

Conformément à la figure 1 il sera maintenant décrit une première variante du procédé de confection d'un ligament prothétique 1 destiné au remplacement d'un ligament articulaire naturel tel que par exemple les ligaments croisés antérieurs ou postérieurs du genou. Un tel ligament prothétique 1 comprend deux parties extrêmes 2,2' normalement destinées à être enchâssées dans les tunnels d'insertion osseuses de l'articulation ; autour des parties extrêmes 2 et 2' une partie médiane 3 est exclusivement formée par un écheveau de fils actifs 4 ; les fils actifs 4 avantageusement en une matière du type polyester biocompatible peuvent être soit mono-filamentaires soit multi-filamentaires, c'est-à-dire formé d'un certain nombre de filaments continus et disposés parallèlement les uns aux autres pour constituer un seul fil 4 dont l'épaisseur doit être la plus faible possible tout en restant suffisante pour supporter les efforts auxquels chaque type de ligament est soumis ; compte tenu des essais qui ont été menés, les meilleurs résultats ont été obtenus avec des fils polyester titrant au maximum 1200 décitex comme par exemple les fils référencés PES TYPE 156 de la société Rhône Poulenc.

Dans toute la suite, la partie médiane 4 du ligament 1 correspond très exactement à la partie intra-articulaire de l'articulation que l'on restaure, par exemple le genou;

Les fils actifs 4 constituant la partie médiane 3 du ligament 1 sont maintenus en leurs extrémités supérieure et inférieure par un assemblage de fils de trame 5,5' les liant transversalement ; les assemblages de fils 4,5,5' constituent les zones extrêmes 2 et 2' de la partie intra-osseuse du ligament 1.

Une telle réalisation est d'ailleurs connue par le brevet français FR-2.688.690 déjà cité dans le préambule.

Selon une première variante du procédé conforme à l'invention, le ligament 1 est confectionné pour présenter naturellement une zone médiane active tordue sur elle-même d'un angle α idéalement égal à 45° ou supérieur, comme représenté schématiquement sur la figure 1 par des repères A et B, alignés verticalement sur la partie gauche de la figure 1 montrant en développé la confection de la laize 6 telle qu'elle sera décrite plus loin et décalés d'un angle α sur la partie droite montrant un ligament terminé.

Compte tenu de ce que les fils actifs 4 sont libres transversalement dans la partie médiane 3 du ligament, ce dernier ne peut présenter naturellement de torsion A,B. Dans ces conditions, il est proposé selon l'invention de donner à chaque fils actifs 4 au moment de la préparation de la laize 6, une torsion longitudinale de même sens et de même valeur. Pour donner cette torsion à chaque fil actif 4 il est procédé par déroulement axial desdits fils 4 par leur extrémité libre 7 à partir de bobines fixes 8 dont le sens originel de bobinage déterminera finalement le sens de torsion de chaque fil 4.

On comprend en effet que, les bobines 8 distribuant les fils 4 étant fixes, si l'on tire verticalement en maintenant sans compensation l'extrémité 7 de chaque fil 4, celui-ci se vrille sur lui-même suivant une hélice dont le pas coïncide avec une spire de la bobine, le diamètre de celle-ci déterminant la torsion angulaire par unité de longueur.

On voit bien dès lors que l'on peut aisément régler le couple de torsion de chaque fil 4 en choisissant le sens d'enroulement du fil sur chaque bobine 8 dont le diamètre par ailleurs déterminera le couple final de torsion.

L'ensemble des fils 4 ainsi entournés étant alors disposés parallèlement à eux-mêmes pour former la chaîne de la laize 6, il suffira d'une manière tout à fait connue d'opérer un assemblage de ces fils 4 par des fils transversaux 5,5' avantageusement de même qualité pour former la trame de la laize 6 du moins sur deux bandes latérales correspondant aux zones d'extrémités osseuses 2,2' du ligament telles que montrées à gauche de la figure 1 ; il sera tout à fait aisé de confectionner la laize 6 suivant toutes techniques que maîtrise parfaitement l'homme du métier, par exemple le tissage, le tricotage, le tressage, le couturage ou analogue, etc ..., de telle manière que la même laize 6 roulée sur elle-même à partir d'une de ses lisières 9 ou 10 procure un ligament 1 à fils libres 4 correspondant à l'espace central de la laize 6 compris entre les deux bandes latérales tramées.

Naturellement, plusieurs variantes sont encore possibles pour la confection d'un ligament par roulage convolute de la laize 6 : on peut en effet obtenir un ligament 1 à un seul faisceau par simple roulage à partir soit de la lisière 9 soit de la lisière 10 de la laize 6 en spires concentriques dont la dernière est couturée longitudinalement à la précédente sur ces deux parties extrêmes 2 et 2', tel que représenté en figure 3a et 3b ; on peut également former le ligament 1 par roulage convolute à enroulement inversé à partir de chaque lisière 9,10 les deux faisceaux en contact étant couturés longitudinalement sur les deux parties extrêmes 2 et 2' procurant un ligament à deux axes longitudinaux plus proche de l'anatomie, comme représenté schématiquement sur la figure 3c.

Quelque soit le roulage de la laize 6 il est clair que la torsion interne donnée à chacun des fils actifs 4 développera sur la partie médiane 3 du ligament 1 un couple de même valeur et de même sens sur tous les fils libres de sorte que le ligament aura naturellement un vrillage sur lui-même. Bien entendu il sera alors possible en inversant le sens d'enroulement des bobines 8 au moment de la fabrication de la laize 6, de fabriquer des ligaments 1 dont la torsion naturelle sera droite (figures 3a et 3c) ou gauche (figure 3b).

Cette particularité essentielle des ligaments conformes à l'invention va permettre, comme on l'a vu, le remplacement quasi idéal d'un ligament biologique rompu par une prothèse très proche de l'anatomie. En effet, on a déjà dit, par exemple, que le ligament croisé antérieur subit une torsion d'environ un huitième de tour lorsque le genou passe de sa position maximale de flexion à sa position maximale d'extension. Or, les ligaments prothétiques croisés antérieurs sont généralement implantés lorsque le genou est en flexion maximale. Il est donc tout à fait avantageux d'implanter un ligament pré-contourné en hélice, conformément au procédé détaillé ci-avant, procurant des qualités biomécaniques de stabilité et d'isométrie presque idéales. En outre, ce type de ligament dont la partie médiane active 3 est constituée de fils longitudinaux 4 non liés entre-eux transversalement pré-orientés selon en enroulement intrinsèque droit ou gauche, est le seul qui permette non seulement d'éviter les contraintes en torsion que subissent les ligaments usuels et qui sont la cause majeure de leur rupture, mais encore la production du phénomène de recrutement progressif des fibres lors de la mise en tension brutale du ligament. Des tests mécaniques en laboratoire effectués sur des machines reproduisant la cinétique du genou ont montré que les ligaments tels que décrits dans la présente invention ont une résistance à la fatigue très supérieure à celle des ligaments connus antérieurement y compris ceux décrits par le demandeur dans ses brevets FR-2.697.151 et FR-2.688.690.

Selon une autre variante du procédé conforme à l'invention, pour obtenir une torsion droite ou gauche d'ailleurs, des fils actifs il peut être avantageux de câbler deux fils 11,12 (chacun pouvant être formés d'un seul ou de plusieurs filaments comme il a été dit) , montés en torsion, c'est-à-dire torsadés sur eux-mêmes dans un sens ou dans un autre, conformément à la représentation schématique de la figure 2 où l'on a représenté une torsion S dans le sens horaire et une torsion Z dans le sens anti-horaire. On comprend bien que chaque câblé présente une torsion naturelle dans un sens ou dans l'autre ; il suffit alors de remplacer les fils actifs 4 du procédé précédent par un câblé 13 pour obtenir de la même manière une laize 6 qui, roulée sur elle-même à partir d'une ou de ses deux lisières, formera un ligament conforme à l'une ou l'autre des représentations de la figure 3.

Ici encore les parties extrêmes intra-osseuses 2 et 2' du ligament prothétique 1 correspondent comme on l'a vu à la partie tricotée, tissée, tressée, couturée ou analogue de la laize 6 au moyen de fils de trame 5,5' ; de même, la partie médiane 3 du ligament 1 obtenu avec des câblés 13 libres transversalement et parallèles entre eux correspondra exactement à la partie intra-articulaire de la prothèse une fois implantée dans l'articulation.

A cet égard, il est remarquable d'observer que l'on pourra à partir des procédés de fabrication conforment à l'invention, obtenir des ligaments tout à fait adaptés à la plastie ligamentaire envisagée : on peut par exemple prévoir que la longueur de la zone médiane 3 du ligament prothétique 1, qu'elle soit réalisée par un écheveau de fils 4 ou de câblés 13, soit de 30 mm pour servir de ligament croisé antérieur prothétique, ou de 45 mm pour servir de ligament croisé postérieur.

Bien entendu, pour chaque type de prothèse ligamentaire, on devra en outre sélectionner le ligament correspondant à une articulation droite ou à une articulation gauche, ressortant comme on l'a dit, du sens de torsion horaire ou anti-horaire des fils actifs 4 ou des câblés 13.

Il est bien évident que tout autre procédé visant à donner une torsion soit aux câblés 13 soit aux fils actifs 4 entrerait dans le cadre du procédé de confection de ligaments prothétiques pré-orientés conformes à la caractéristique principale de l'invention.

## Revendications

1. Procédé de confection d'un ligament prothétique pour le remplacement d'un ligament articulaire naturel, comprenant entre deux parties extrêmes (2,2') une partie médiane (3) seulement formée par un écheveau de fils actifs (4,11,12) avantageusement en polyester, mono ou multi-filamentaires dont le titre est inférieur ou égal à 1200 décitex, longitudinaux, adjacents et non transversalement liés entre-eux **caractérisé en ce que** préalablement au montage du ligament (1) on donne une torsion longitudinale à chaque fil actif (4,11,12) dont on choisit le sens de torsion, horaire ou anti-horaire, pour former respectivement un ligament prothétique (1) pour articulation droite ou articulation gauche, les extrémités desdits fils (4,11,12) étant maintenues ensemble par tous moyens appropriés sur une longueur suffisante pour constituer les parties extrêmes (2,2') dudit ligament prothétique (1).

2. Procédé selon la revendication précédente ***caractérisé* en ce qu'**on imprime une torsion à chaque fil actif (4,11,12) tel que le couple de torsion qui en résulte crée un vrillage naturel d'au moins un huitième de tour entre les deux extrémités de la partie médiane (3) du ligament (1).

3. Procédé selon l'une quelconque des revendications 1 ou 2 ***caractérisé* en ce que** la torsion longitudinale que l'on donne à chaque fil actif (4) est de même sens et de même valeur.

4. Procédé selon l'une quelconque des revendications précédentes utilisant des fils (4) mono-filamentaires ou composés de plusieurs filaments parallèles, ***caractérisé* en ce que** la torsion de chaque fil actif (4) est obtenue par déroulement axial du fil (4) par son extrémité libre (7) à partir d'une bobine fixe (8) dont le sens de bobinage déterminera le sens de torsion.

5. Procédé selon l'une quelconque des revendications 1 ou 2 ***caractérisé* en ce que** les fils actifs (11,12) sont câblés deux à deux.

6. Procédé selon la revendication précédente ***caractérisé* en ce que** chaque câblé (13) est monté en torsion c'est-à-dire que les fils constitutifs (11,12) sont torsadés en hélice gauche ou droite pour procurer au câblé (13) une torsion naturelle (S,Z) soit dans le sens horaire (S) soit anti-horaire (Z), déterminant respectivement un ligament pour articulation droite ou articulation gauche.

7. Ligament prothétique lévogyre ou dextrogyre pour le remplacement d'un ligament naturel articulaire confectionné par roulage ou pliage convolute et couturage d'une laize (6) apte à maintenir en leurs extrémités (2,2') des câblés (13) ou des fils actifs (4), longitudinaux montés en torsion selon l'une quelconque des revendications 1 à 6 ***caractérisé* en ce que** au moins sur une longueur donnée délimitant la zone médiane (3) du ligament (1), lesdits fils (4) ou lesdits câblés (13) sont adjacents et non transversalement liés entre-eux.

8. Ligament selon la revendication 7 ***caractérisé* en ce que** les parties extrêmes (2,2') de la laize (6) sont constituées par un assemblage formant une trame (5,5') pour le maintien transversal des câblés (13) ou des fils actifs (4) longitudinaux, du type d'un tricotage, tissage, tressage, couturage ou analogue.

9. Ligament selon la revendication précédente ***caractérisé* en ce que** les parties extrêmes (2,2') de la laize (6) comportant un assemblage de trame sont de longueur suffisante pour être enchâssées dans les tunnels d'insertion osseuse de l'articulation et **en ce que** la zone médiane (3) comportant les câblés (13) ou les fils (4) transversalement libres est avantageusement de même longueur que la partie intra-articulaire du ligament naturel (1).

10. Ligament selon la revendication 9 ***caractérisé* en ce que** la longueur de la zone médiane (3) est de 30 mm pour servir de ligament croisé antérieur prothétique pour genou gauche ou droit.

11. Ligament selon la revendication 9 ***caractérisé* en ce que** la longueur de la zone médiane (3) est de 45 mm pour servir de ligament croisé postérieur prothétique pour genou gauche ou droit.

12. Ligament selon l'une quelconque des revendications 7 à 11 ***caractérisé* en ce que** la laize (6) est roulée de façon convolute à partir d'une de ses lisières latérales (9,10) en spires concentriques dont la dernière est couturée longitudinalement à la précédente sur ses deux parties extrêmes (2,2') comportant un assemblage de trame (5,5').

13. Ligament selon l'une quelconque des revendications 7 à 11 ***caractérisé* en ce que** la laize (6) est roulée de façon avantageusement symétrique en double faisceaux convolutes à enroulements inversés à partir de chacune de ses lisières latérales (9,10), les deux faisceaux en contact étant couturés longitudinalement sur ses deux parties extrêmes (2,2') comportant un assemblage de trame (5,5').

## Claims

1. Method of manufacturing a prosthetic ligament for the replacement of a natural articular ligament, comprising between two end parts (2, 2') a median part (3) solely formed by a hank of active yarns (4, 11, 12) advantageously made of polyester, which are mono or multi-filamentary, of which the count is less than or equal to 1200 decitex, and which are longitudinal, adjacent and not transversely connected together, **characterized in that**, prior to the assembly of the ligament (1), a longitudinal torsion is given to each active yarn (4, 11, 12), of which the direction of torsion, clockwise or .anti-clockwise, is chosen in order to form a prosthetic ligament (1) for right joint or left joint respectively, the ends of said yarns (4, 11, 12) being held together by any appropriate means over a length sufficient to constitute the end parts (2, 2') of said prosthetic ligament (1).

2. Method according to the preceding Claim, **characterized in that** a torsion is imparted to each active yarn (4, 11, 12) such that the resulting torsional moment creates a natural twist of at least an eighth of turn between the two ends of the median part (3) of the ligament (1).

3. Method according to either one of Clams 1 or 2, **characterized in that** the longitudinal torsion which is given to each active yarn (4) is of the same direction and of the same value.

4. Method according to any one of the preceding Claims using yarns (4) which are mono-filamentary or composed of a plurality of parallel filaments, **characterized in that** the torsion of each active yarn (4) is obtained by axial unwinding of the yarn (4) by its free end (7) from a fixed bobbin (8) of which the direction of winding will determine the direction of torsion.

5. Method according to either one of Claims 1 or 2, **characterized in that** the active yarns (11, 12) are cabled in twos.

6. Method according to the preceding Claim, **characterized in that** each cable (13) is mounted in torsion, i.e. the constituent yarns (11, 12) are twisted in a left-hand or right-hand helix in order to procure for the cable (13) a natural torsion (S, Z) either in clockwise (S) or anti-clockwise direction (Z), respectively determining a ligament for right joint or left joint.

7. Laevo-rotatory or dextro-rotatory prosthetic ligament for the replacement of a natural articular ligament, manufactured by convolute rolling or folding and seaming of a width (6) adapted to hold at their ends (2, 2') longitudinal cables (13) or active yarns (4) mounted in torsion, according to any one of Claims 1 to 6, **characterized in that**, at least over a given length delimiting the median zone (3) of the ligament (1), said yarns (4) or said cables (13) are adjacent and not transversely connected together.

8. Ligament according to Claim 7, **characterized in that** the end parts (2, 2') of the width (6) are constituted by an assembly forming a weft (5, 5') for the transverse hold of the cables (13) or of the longitudinal active yarns (4), of the knitting, weaving, braiding, seaming or the like type.

9. Ligament according to the preceding Claim, **characterized in that** the end parts (2, 2') of the width (6) comprising a weft assembly are of sufficient length to be fitted in the osseous insertion tunnels of the joint and **in that** the median zone (3) comprising the cables (13) or the transversely free yarns (4) is advantageously of the same length as the intra-articular part of the natural ligament (1).

10. Ligament according to Claim 9, **characterized in that** the length of the median zone (3) is 30 mm in order to serve as prosthetic anterior cruciate ligament for left or right knee.

11. Ligament according to Claim 9, **characterized in that** the length of the median zone (3) is 45 mm in order to serve as prosthetic posterior cruciate ligament for left or right knee.

12. Ligament according to any one of Claims 7 to 13, **characterized in that** the width (6) is wound in convolute manner from one of its lateral edges (9, 10) in concentric turns of which the last is seamed longitudinally to the preceding one on its two end parts (2, 2') comprising a weft assembly (5, 5').

13. Ligament according to any one of Claims 7 to 11, **characterized in that** the width (6) is wound in advantageously symmetrical manner in double convolute bundles with inverted windings from each of its lateral edges (9, 10), the two bundles in contact being seamed longitudinally on its two end parts (2, 2') comprising a weft assembly (5, 5').

## Patentansprüche

1. Verfahren zur Anfertigung eines prothetischen Ligaments als Ersatz für ein natürliches Gelenkligament, umfassend zwischen zwei Endteilen (2,2') einen Mittelteil (3), der nur von einem Strang von längs verlaufenden, benachbarten und untereinander nicht quer verbundenen aktiven Fäden (4,11,12) aus einem oder mehreren Filamenten, vorteilhafterweise aus Polyester, gebildet wird, deren Titer kleiner oder gleich 1200 Dezitex ist, ***dadurch gekennzeichnet*, dass** man vor der Anbringung des Ligaments (1) jedem aktiven Faden (4,11,12) einen Längsdrall gibt, dessen Drehrichtung, im Uhrzeigersinn oder entgegen dem Uhrzeigersinn, man wählt, um ein prothetisches Ligament (1) für ein rechtes Gelenk bzw. ein linkes Gelenk zu bilden, wobei die Enden der Fäden (4,11,12) durch jegliche geeigneten Mittel auf einer ausreichenden Länge zusammengehalten werden, um die Endteile (2,2') des prothetischen Ligaments (1) zu bilden.

2. Verfahren nach dem vorangehenden Anspruch, ***dadurch gekennzeichnet*, dass** man jedem aktiven Faden (4,11,12) einen solchen Drall verleiht, dass das Drehmoment, das daraus resultiert, eine natürliche Verwindung von mindestens einer achtel Umdrehung zwischen den zwei Enden des Mittelteils (3) des Ligaments (1) erzeugt.

3. Verfahren nach einem der Ansprüche 1 oder 2, ***dadurch gekennzeichnet,* dass** der Längsdrall, den man jedem aktiven Faden (4) gibt, von gleicher Richtung und gleicher Größe ist.

4. Verfahren nach einem der vorangehenden Ansprüche, unter Verwendung von Fäden (4), die aus einem Filament bestehen oder aus mehreren parallelen Filamenten zusammengesetzt sind, ***dadurch gekennzeichnet*, dass** der Drall von jedem aktiven Faden (4) durch axiales Abwickeln des Fadens (4) mittels seines freien Endes (7) von einer festen Spule (8) erhalten wird, deren Wicklungsrichtung die Drehrichtung bestimmen wird.

5. Verfahren nach einem der Ansprüche 1 oder 2, ***dadurch gekennzeichnet*, dass** die aktiven Fäden (11,12) paarweise verseilt sind.

6. Verfahren nach dem vorangehenden Anspruch, ***dadurch gekennzeichnet*, dass** jede Verseilung (13) mit einem Drall angebracht wird, das heißt, dass die konstituierenden Fäden (11,12) in einer Links- oder Rechtsschraube verdrillt sind, um der Verseilung (13) einen natürlichen Drall (S, Z) entweder im Uhrzeigersinn (S) oder entgegen dem Uhrzeigersinn (Z) zu verschaffen, womit ein Ligament für ein rechtes Gelenk bzw. ein linkes Gelenk festgelegt wird.

7. Linksdrehendes oder rechtsdrehendes prothetisches Ligament als Ersatz für ein natürliches Gelenkligament, gefertigt durch Aufrollen oder rollenartiges Falten und Vernähen von einer Bahnbreite (6), welche geeignet ist, der Länge nach verlaufende Verseilungen (13) oder aktive Fäden (4), die gemäß einem der Ansprüche 1 bis 6 mit einem Drall angebracht werden, an ihren Enden (2,2') zu halten, ***dadurch gekennzeichnet,* dass** die besagten Fäden (4) oder die besagten Verseilungen (13) mindestens auf einer gegebenen, die Mittelzone (3) des Ligaments (1) begrenzenden Länge benachbart und untereinander nicht quer verbunden sind.

8. Ligament nach Anspruch 7, ***dadurch gekennzeichnet,* dass** die Endteile (2,2') der Bahnbreite (6) für den Querzusammenhalt der der Länge nach verlaufenden Verseilungen (13) oder aktiven Fäden (4) von einem einen Schuss (5,5') bildenden Verband von der Art eines Gewirks, Gewebes, Geflechts, Nähstoffs oder dergleichen gebildet werden.

9. Ligament nach dem vorangehenden Anspruch, ***dadurch gekennzeichnet*, dass** die Endteile (2,2') der Bahnbreite (6) mit einem Schussverband von ausreichender Länge sind, um in die Knocheneinführkanäle des Gelenks eingepasst zu werden, und dass die Mittelzone (3), welche die in Querrichtung freien Verseilungen (13) oder Fäden (4) aufweist, vorteilhaft von derselben Länge ist, wie der intraartikuläre Teil des natürlichen Ligaments (1).

10. Ligament nach Anspruch 9, ***dadurch gekennzeichnet,* dass** die Länge der Mittelzone (3) 30 mm beträgt, um als prothetisches anteriores Kreuzband für ein linkes oder rechtes Knie zu dienen.

11. Ligament nach Anspruch 9, ***dadurch gekennzeichnet*, dass** die Länge der Mittelzone (3) 45 mm beträgt, um als prothetisches posteriores Kreuzband für ein linkes oder rechtes Knie zu dienen.

12. Ligament nach einem der Ansprüche 7 bis 11, ***dadurch gekennzeichnet*, dass** die Bahnbreite (6) von einem ihrer seitlichen Ränder (9,10) her in rollenartiger Weise in konzentrischen Windungen aufgewickelt ist, von denen die letzte an seinen beiden Endteilen (2,2') mit einem Schussverband (5,5') der Länge nach mit der vorangehenden vernäht ist.

13. Ligament nach einem der Ansprüche 7 bis 11, ***dadurch gekennzeichnet*, dass** die Bahnbreite (6) von jedem ihrer seitlichen Ränder (9,10) her vorteilhaft in symmetrischer Weise in rollenartigen Doppelbündeln mit umgekehrter Aufrollrichtung aufgewickelt ist, wobei die beiden im Kontakt befindlichen Bündel an seinen beiden Endteilen (2,2') mit einem Schussverband (5,5') der Länge nach vernäht sind.
